# EUROPEAN PATENT APPLICATION

(11) **EP 4 609 809 A2**
(43) Date of publication of application: **03.09.2025**
(21) Application number: 25156742.6
(22) Date of filing: 10.02.2025
(51) Int. Cl.: A61B 17/74

(54) **TROCHANTERIC NAILING SYSTEMS**

(30) Priority: 08.02.2024 US 202463551305 P; 29.04.2024 US 202418648694
(71) Applicant: Globus Medical, Inc., Audubon, PA 19403 (US)
(72) Inventor: GLERUM, Chad, PENNSBURG, 18073 (US); YUCHIMIUK, Nathaniel, SANATOGA, 19464 (US); PETERSON, John, CONSHOHOCKEN, 19428 (US)
(74) Representative: Morabito, Sara

(57) **Abstract**

A trochanteric nailing system makes use of a lag screw operatively connectable to a trochanteric nail. The lag screw has a helical thread disposed over a suitable surgical length on the outer surface of the shaft of the lag screw. The helical thread includes undercuts formed therein to improve resistance to cutout or lag screw migration. The lag screw may also include a variable-width thread to improve fixation and resistance to migration or cut out. Helical flutes reduce torque forces exerted on bone during insertion, while maintaining the improved fixation of other features about the entire circumference of the lag screw. The lag screw may also be equipped with tapered set screw grooves which engaging opposing portions of a set screw of the trochanteric nail when received therein to resist migration or cutout.

## Description

### CROSS-REFERENCE TO RELATED APPLICATIONS

This application is a non-provisional application claiming the benefit of priority of Provisional Patent App. 63/551,305, filed February 8, 2024.

### FIELD

This disclosure relates generally to the orthopedic surgical field and to trochanteric nailing systems, in particular.

### BACKGROUND

The significant long bones of the extremities are the humerus, radius and ulna of the upper extremity and the femur and tibia of the lower extremity. Following an injury to the long bone, and in particular, injuries resulting in one or more fractures of the long bone, one or more fixation devices may be used to immobilize the fracture fragments and stabilize the long bone. Bone fractures can be treated with screws or other fixation devices inserted into or through the bone to stabilize it once the fractured portions have been brought into proper alignment. Femoral neck fixation, for example, can be used to treat hip fractures by inserting an intramedullary nail into the medullary cavity of the fractured femur followed by insertion of a fixation screw into the femoral neck/head at an angle relative to the intramedullary nail. Similarly, other long bone fractures can be treated by inserting an intramedullary nail into the intramedullary canal of the bone and providing the appropriate proximal and/or distal fixation. Trochanteric (or hip) nailing systems traditionally employ a single, large diameter (~10mm) lag screw placed through an intramedullary nail and into the femoral head to fix hip fractures. Such systems often suffer from various drawbacks and disadvantages. One potential complication is referred to as cutout, which may be described as an extrusion of the lag screw from the femoral head as a result of cranial migration of the screw and varus collapse of the neck-shaft angle. It would be desirable to address these drawbacks, as well as foster potential improvements in trochanteric nailing systems.

### SUMMARY

According to the invention it is provided a trochanteric nailing system according to claim 1. Further advantageous aspects of the invention are set forth in the dependent claims. In one possible implementation, a trochanteric nailing system includes a trochanteric nail and an associated trochanteric nail fixation device. The trochanteric nail fixation device has a lag screw which is designed to be rotatably advanced into a femur which is the subject of a procedure associated with the trochanteric nailing system. A helical thread extends on the shaft of the lag screw at its forward end. The thread has a threadform into which at least one undercut has been formed. The presence of the undercut enables the lag screw to retain, grab or otherwise engage bone matter within or adjacent the undercut. By such engagement of bone matter, when the lag screw is subject to a lateral force, during post-operative patient activity, for example, the bone matter held within the undercut on whatever side of the lag screw is being subjected to tension from the lateral force, acts to increase resistance against such lateral force. In this way, the undercut portions placed under tension from a lateral force, with bone matter received therein, help hold the lag screw against undesired migration.

In another implementation, the undercut is formed on both the leading and the trailing edges of the thread and the undercut extends over a predetermined surgical length associated with the thread.

In still further implementations, the thread form of the thread may be designed with a variable, cross-sectional width over adjacent courses of the thread, with the width of the thread increasing from the tip toward the head of the shaft. This configuration results in increased compression of the bone matter as the lag screw is inserted.

Certain implementations of the trochanteric nailing system have at least one flute defined in the lag screw. The thread of the lag screw has a first pitch, whereas the flute extends helically with a second pitch. The second pitch for the flute is coarser, that is, less fine, than the first pitch of the thread, which means the flute crosses the thread to define channels extending through the sides of the thread at different corresponding radial locations.

In certain implementations, there are three of such helical flutes having starting locations at different, angularly spaced locations about the shaft of the lag screw.

Still other implementations of the trochanteric nailing system include a bore extending through the trochanteric nail at an angle appropriate for the associated hip procedure. The bore is sized to receive the lag screw therethrough during the hip procedure. The lag screw is locked either dynamically or statically in relation to the trochanteric nail by locking a set screw of the trochanteric nail into an opposing set screw groove defined in the lag screw shaft. The lag screw has one or more of such set screw grooves designed with a taper in the distal direction, with the result that engagement by the lag screw with the set screw of the trochanteric nail is increased.

A method of manufacturing a lag screw is provided in certain implementations of this disclosure. The method involves forming a threadform differently from a more typical V shaped threadform of the lag screw so that the threadform includes concavities to define undercuts on both the leading and trailing edges of the thread. The lag screw is further manufactured such that it has a variable width thread while maintaining a constant average pitch. The manufacturing process may also involve increasing the widths of successive courses of the thread from the tip toward the proximal end to increase compression as the lag screw is rotatably inserted into bone matter during the hip procedure.

### BRIEF DESCRIPTION OF THE DRAWINGS

A more complete understanding of the present invention, and the attendant advantages and features thereof, will be more readily understood by reference to the following detailed description when considered in conjunction with the accompanying drawings, wherein:
Fig. 1 is a cross-sectional view of one implementation of a trochanteric nailing system according to the present disclosure;
Fig. 2 is an enlarged, cross sectional, partial view of a lag screw of a trochanteric nailing system according to one possible implementation of this disclosure;
Fig. 3 is a partially schematic, cross-sectional view of the lag screw of Fig. 2 of the present disclosure;
Fig. 4 is a side elevational view of the lag screw of Figs. 2 and 3 showing bone matter after insertion into a femur;
Fig. 5 is a schematic view of a cross section of another possible implementation of the thread of a lag screw of the present disclosure;
Fig. 6 is an enlarged, side elevation view of a tip of a lag screw according to an implementation of the present disclosure;
Fig. 7 is an enlarged, endview of the tip of the lag screw shown in Fig. 6; and
Fig. 8 is an enlarged, cross-sectional view of a proximal portion of the lag screw shown in Fig 1.

### DETAILED DESCRIPTION

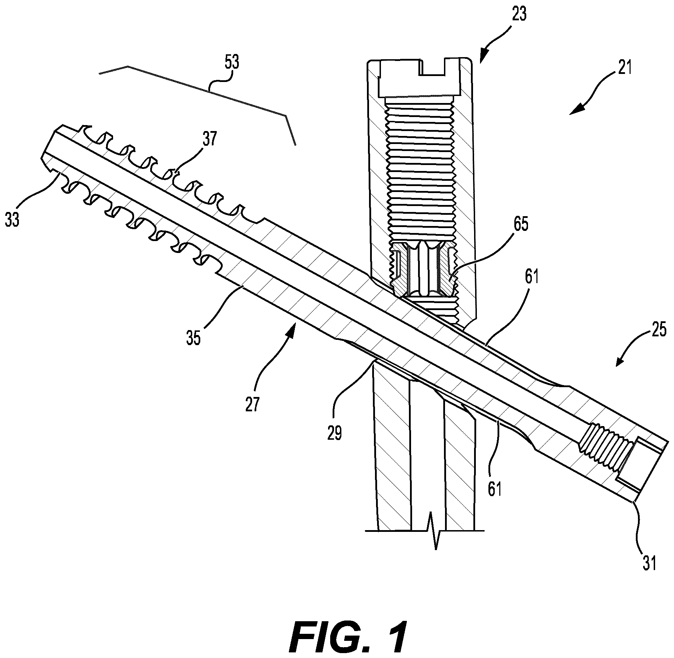

Referring now to Figs 1-6, a trochanteric nailing system 21 makes use of a trochanteric nail 23 which is configured for intramedullary insertion into a femur of a patient to address a hip fracture, dislocation or other condition during an associated surgical procedure. Nailing system 21 likewise includes a trochanteric nail fixation device 25 which is designed to be suitably connected to trochanteric nail 23. In the illustrated implementation, trochanteric nail fixation device 25 includes a lag screw 27 which is configured to be rotatably advanced into the femur and engage bone matter adjacent to lag screw 27 in response to torque applied to lag screw 27. As illustrated, lag screw 27 passes through a suitably angled bore 29 extending diametrically through the shaft of trochanteric nail 23.

Lag screw 27 has a head 31 and the tip 33 located at opposite ends of lag screw 27, and a shaft 35 extending between head 31 and 33. A helical thread 37 is disposed on the outer surface of shaft 35 at the distal end of lag screw 2. Thread 37 has a thread start at tip 33 and winds proximally from tip 33 partially up shaft 35 toward head 31. The relative longitudinal extent of thread 37 relative to shaft 35 may be varied to suit any number of operational parameters within the scope of this disclosure.

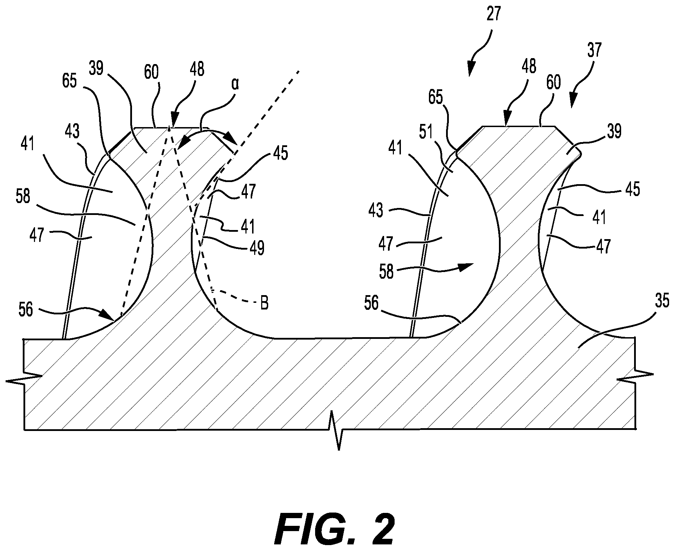

Referring more particularly to Fig. 2, thread 37 has its threadform 39 shown in cross section with a concavity 41 defined in one or both of the leading and trailing edges 43, 45, respectively. In the illustrated embodiment, concavities 41 are defined in both leading and trailing edges, such concavities being referred to herein, respectively, as undercuts 47.

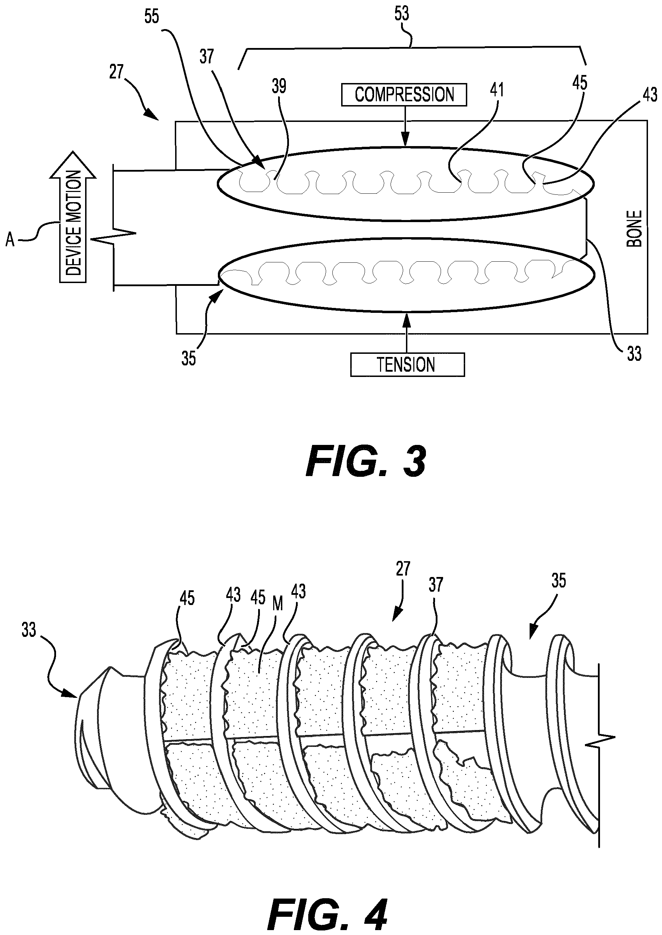

Thread 37 is designed in this implementation to have a predetermined pitch selected relative to the bone matter through which it will be passing so that bone matter M (Fig. 4) is able to be received and engaged within undercuts 47. By way of further illustration with reference to Figs. 2 and 4, bone matter M (Fig. 4) is received between opposing pairs of undercuts 47 on leading and trailing edges 43, 45 of adjacent, longitudinally spaced, thread portions 48 (Fig. 2). Bone matter M thus is engaged by undercuts 47, which undercuts are extending longitudinally along lag screw 27 over a surgical length 53 suitable for the intended procedure. Two of such adjacent thread portions 48 are shown by way of example by reference numerals 49, 51 in Fig. 2. Each 360° arc of thread 37 shall be referred to herein as a respective thread courses).

Referring now more particularly to Figs 3 and 4, after fixation of trochanteric nailing system 21 to the femur, the system periodically becomes subject to lateral force during patient activity, such as that shown by arrow A (Fig. 3). In response to such lateral force on lag screw 27, bone matter M located in portions of undercuts 41 opposite such force will resist such lateral force, akin to the bone matter and the corresponding undercut opposing separation from each other and thereby exerting a retaining or tensile force upon lag screw 27 along that side or half of shaft 35 under tension. As such, a tensile force is created by the aforementioned portions of undercuts 41, such tensile forcing acting to oppose the lateral force induced by patient activity on nailing system 21. Such tensile force adds to the resistance caused by compression of the bone matter on the side of the lag screw 37 which is being subject to the lateral force.

In addition to the above-described tensile force, formation of undercuts 47 along surgical length 53 of lag screw 27 creates a lateral profile 37 and its corresponding surface area along surgical length 53 (Fig. 3). Such lateral profile is greater than a comparable thread form without undercuts 47, such as a thread form with a simple pointed crest and a V shape between adjacent lateral sides of the thread. Otherwise stated, the addition of undercuts 47 and their corresponding concavities 41 increase the effective surface area of the surgical length 53 by virtue of adding the arcuate surfaces associated with concavities 41, instead of planar surfaces which may be otherwise associated with a threadform.

By way of example only, referring to the implementation illustrated in Fig. 2, the concavities of undercuts 47 may define an angle α of at least 45 degrees measured from an associated reference triangular threadform indicated by reference letter B. Other values for angle α are likewise suitable, as are other shapes or configurations of undercuts 47, which would result in increased surface area of lateral profile 55 over surgical length 53. The increase in lateral profile surface area acts to lower stress concentrations, lower force per unit area, and otherwise improve distribution of force which may be exerted on one or both sides of shaft 35 post operatively, thereby potentially resisting undesirable lag screw migration or cutout.

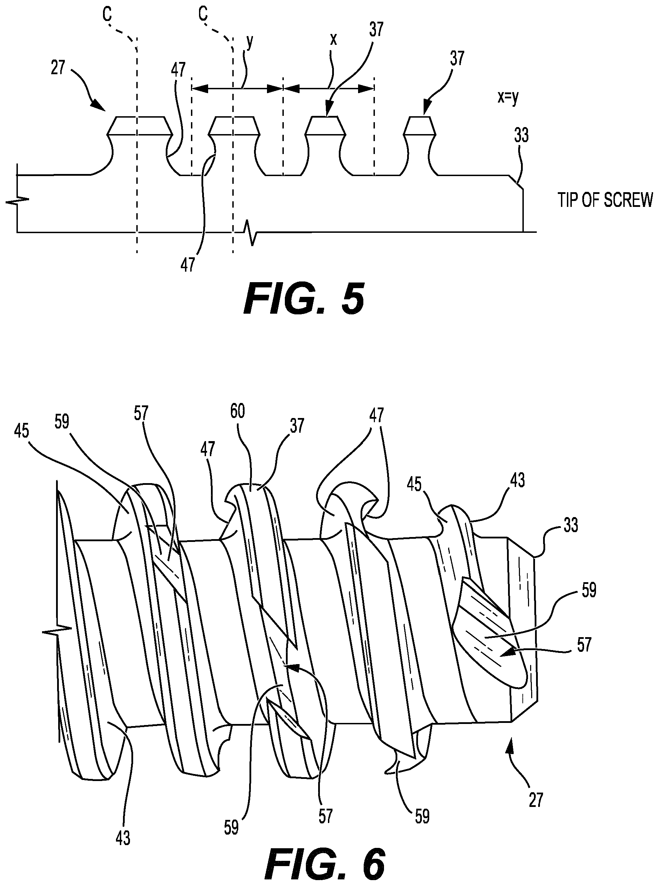

Referring now more particularly to Fig 5, threadform 39 is characterized by a centerline shown by reference arrow C. In one possible implementation, thread 37 and its threadform 39 are characterized by a variable, cross-sectional width shown by reference lines W over adjacent courses of thread 37. Width W of thread 37 may gradually increase from tip 33 as thread 37 winds toward head 31. Such configuration produces the biomechanical effect of increasing compression relative to the bone matter being acted upon by lag screw 27 during insertion thereof. Defining pitch just for Fig. 5 as being centerpoints labeled x and y between undercuts 47 of adjacent courses, even though width W of thread 37 varies as it travels from tip 33 to head 31, the distance between centerpoints x and y, that is, the pitch as defined for this implementation, remains constant.

In addition, or alternately, a variable width profile as discussed above for thread 37 may be achieved by ascribing a different pitch to the leading and trailing edges, with the pitches selected to increase the distance between the leading and trailing edges from each other as the thread winds toward the head from the tip. The resulting variable width threadform would likewise produce the desired biomechanical increased compression upon surgical insertion.

Thread 37 may be further defined as having a root 56 located radially proximal to the outer surface of shaft 35. Extending from root 56 are two, laterally extending thread sides 58 terminating in a crest 60 of thread 37. Root 56 and Crest 60 correspond to the minor and major diameters characteristic of thread 37. Thread sides 58 correspond, respectively, to leading or trailing edges 43, 45 of thread 37.

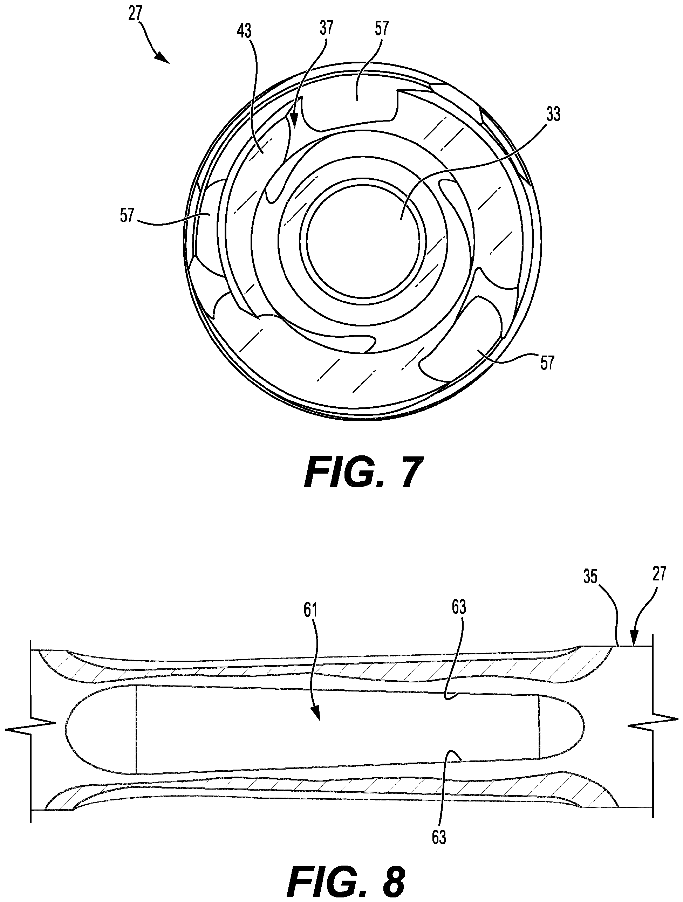

Referring more particularly to Figs. 6 and 7, at least one flute 57 is defined in lag screw 27 and is disposed to also extend helically across the same surgical insertion length 53 as thread 37. Flute 57 generally contributes to lessening the amount of torque required for lag screw insertion, especially for self-tapping or similar applications where it is desirable to reduce insertion torque. Flute 57 extends using a second pitch which is coarser than the first pitch of thread 37. As a result, the disposition of flute 57 and its coarser pitch define channels 59 extending through respective thread sides 58, at angular locations which differ between adjacent courses of thread 37. Otherwise stated, channels 59 are staggered at different locations about the circumference of lag screw 27 over several adjacent courses of thread 37, as opposed to what would be the case in a non-helical disposition of flute 57.

Although channels 59 eliminate portions of undercuts 47 and associated potential benefits of increased fixation, any such potential loss of fixation is distributed to different angular locations about the circumference of shaft 35. As such, flute 57 does not reduce the resistance to lateral migration and resistance to potential cut-out in any one angular position relative to another, maintaining the same resistance to migration regardless of the orientation of lag screw 27 or the direction of a lateral force on lag screw 27.

In the illustrated embodiment, as best seen in Fig. 6, there are three flutes 57 having three corresponding start locations at angularly spaced locations about shaft 35, in this case, evenly distributed about a 360-degree arc. Channels 59 created by flutes 57 in this embodiment do not extend in depth inwardly of root 56. As a result, at least 300 degrees of undercut 47 remains located in each 360-degree arc of thread 37 to engage adjacent bone matter and enhance fixation. Other dimensions of channels 59 are possible. Width of channels 59 may be selected so as to balance the reduced torque required due to flutes 57 with the desired increase in resistance to lag screw migration from undercuts 47.

Referring now more particularly to Fig. 8 in combination with Fig. 1, shaft 35 of lag screw 27 may include four, set screw grooves 61. Grooves 61 have respective pairs of opposing groove sidewalls 63. Screw grooves 61 in the illustrated implementation are defined at evenly spaced angular locations around the cylindrical surface of trochanteric nail 23, in this case, at about 90° intervals. Other configurations may likewise be suitable for other applications.

Undercuts 47 in the illustrated implementation extend in a single, concave arc from a location proximal to root 56 radially outwardly to a portion adjacent to crest 60. Undercut 47 has an outer undercut tip 65 (Fig. 2) formed at or adjacent to crest 60. This and any other geometry in other applications, which receives and retains bone matter in undercuts 47, are suitable.

Still further variations to the disclosed implementations are within the scope of this disclosure. Thread 37 in the illustrated embodiment is shown as a single start thread, but it will be appreciated that the components and features of this disclosure are likewise applicable to double start threads or other thread configurations.

While thread 37 in the illustrated implementation is male, it may be female in other potential applications.

Thread 37 may be configured as either right-handed or left-handed, for appropriate use on a patient's right or left side to minimize potential loss of fracture reduction during insertion.

From the foregoing disclosure, an improved lag screw may thus be manufactured to increase resistance against cut out after a trochanteric hip procedure. The manufacturing would involve forming a threadform with increased surface area as compared to a standard V shaped threadform, and also manufacturing the threadform to include undercuts on one or both of the leading and trailing edges of the thread. In addition, a lag screw can be made with a thread that has a variable profile width, increasing proximally, while making such lag screw with a constant average pitch despite the increasing thread width. A lag screw manufactured in this manner would increase compression as it is rotatably inserted into bone matter during the hip procedure to increase fixation.

The operation and advantages of the trochanteric nailing system 21 are apparent from the foregoing disclosure:

The disclosed system has improved single lag screw fixation to better resist cutout, delaying cranial migration and extending the time available for healing to occur.

The hip nail fixation device incorporates an undercut on the leading and trailing edges of the thread of the lag screw, with both edges wrapping around bone upon insertion.

These undercuts grab the bone radially, meaning that when a lateral force is applied to the device, the bone around the device opposite the direction of migration is put into tension to resist lateral migration, in addition to the bone in compression.

The stress in the bone is effectively spread around the device incurred by a given load as compared to a traditional lag screw and reduces the volume of bone that is subject to loading past its ultimate stress. In short, a traditional screw pushes on bone above the screw to resist cutout. In contrast, the disclosed trochanteric nail system 21 pushes on bone above the screw and pulls on bone below the screw to resist cutout.

In addition, as disclosed, the disclosed threadform and the bone trapped inside the undercuts creates an increased, effective lateral profile of the device beyond that of the device body alone. This additional lateral profile distributes the compressive force over a larger area to resist cutout.

In certain implementations, the geometry of thread 37 itself, in combination with the undercuts 47, has been modified to improve bone performance in tension by applying additional compression to bone trapped within the undercuts during insertion. This additional compression may be gained via a variable thread profile that widens as the thread travels down the body of the device while maintaining a constant pitch at the center of the profile. In addition, or alternately, a variable width profile is formed by using a different pitch for the leading and trailing edges of the thread profile where the average pitch between the two edges is the pitch of the thread.

The helical flute disclosed herein equalizes biomechanical performance in all directions of lateral migration while still providing the advantages of a traditional flute.

The addition of a taper to the set screw groove increases resistance to sliding in the lateral direction via mechanical interference between the set screw in the nail and the disclosed fixation device and its lag screw.

Additionally, the disclosed features reduce risk of complications from potentially high insertion torque (which can lead to rotation of the femoral head and loss of reduction during insertion) via device options that allow for clockwise or counterclockwise insertion. By having both left- and right-handed versions, soft tissue, muscle anatomy, and common fracture patterns better resist clockwise insertion in the right hip and counter-clockwise insertion in the left hip. The likelihood of reduction loss during insertion can be reduced via appropriate clockwise or counterclockwise device selection.

It will be further understood that various changes in the details, materials, and arrangements of the parts which have been described and illustrated in order to explain the nature of this invention may be made by those skilled in the art without departing from the scope of the invention as expressed in the claims. One skilled in the art will appreciate that the embodiments discussed above are nonlimiting. It will also be appreciated that one or more features of one embodiment may be partially or fully incorporated into one or more other embodiments described herein.

The invention could be defined inter alia by the following examples:
1. A trochanteric nailing system, comprising: a trochanteric nail configured for intramedullary insertion into a femur of a patient to address a hip fracture; and a trochanteric nail fixation device operatively connectable to the trochanteric nail, wherein the trochanteric nail fixation device comprises a lag screw configured to be rotatably advanced into the femur and engage bone matter adjacent to the lag screw in response to torque applied thereto, wherein the lag screw has a head and a tip formed at opposite ends of the lag screw and a shaft extending at least partially between the head and the tip; a helical thread disposed on the outer surface of the shaft, extending longitudinally at a distal location from the head to terminate at the tip, the thread having a root located radially proximal to the outer surface of the shaft, the thread further having thread sides extending laterally from the root to terminate in a thread crest, whereby the root and the thread crest corresponding to the minor and major diameters of the thread, the thread sides corresponding, respectively, to leading and trailing edges of the thread; wherein the thread comprises a threadform having a concavity defined in at least one of the leading edge and the trailing edge to form at least one, corresponding undercut; wherein the thread is characterized by a predetermined pitch selected relative to the bone matter to enable engagement of the bone matter adjacent the lag screw by the undercut, whereby, in response to a lateral force in a given force direction on the lag screw after insertion into the femur, the bone matter engages in portions of the undercut located opposite the given force direction to resist the lateral force.
2. The system of Example 1, wherein the threadform comprises two concavities, one on each of the lateral sides of the threadform to create two, respective undercuts on each of the leading edge and the trailing edge.
3. The system of Example 2, wherein the thread is located to extend longitudinally on the shaft over a predetermined surgical insertion length less than the length of the shaft between the head and the tip, and the undercuts extend on the helical thread over the predetermined surgical length.
4. The system of Example 3, wherein the surgical length and the thread extending over the surgical length define a lateral profile oriented to oppose the lateral force when applied to the lateral profile.
5. The system of Example 4, wherein the threadform comprises a centerline and comprises a variable cross-sectional width over adjacent courses of the thread, the width increasing from the tip toward the head of the shaft to increase compression of the bone matter during insertion of the lag screw.
6. The system of Example 4, wherein the threadform is shaped to have a first pitch for the leading edge and a second pitch for the trailing edge, the first and second pitches selected to differ from each other and selected to cause increased compression relative to the bone matter on insertion of the lag screw.
7. The system of Example 1, wherein the threadform is characterized by a first pitch, and wherein the lag screw further comprises at least one flute extending helically with a second pitch, the second pitch being a coarser pitch than the first pitch of the thread to define channels extending through respective thread sides at corresponding radial locations which differ between adjacent courses of the thread.
8. The system of Example 7, further comprising three helical flutes having three corresponding flute starts at angular spaced locations about the shaft.
9. The system of Example 8, wherein the three flutes are defined at three, corresponding arcuately spaced locations in a 360 degree turn of the thread.
10. The system of Example 1, wherein the trochanteric nail further comprises an outer, cylindroid surface characterized by a longitudinal axis and a cross-sectional diameter, the nail having portions defining a bore extending transversely and diametrically through the shaft at an angle to the longitudinal axis, the bore sized to receive the lag screw therethrough, wherein the system further comprises a set screw interconnectable between the nail and the screw, wherein the lag screw is operatively connectable to the trochanteric nail by locking of the set screw to the lag screw after insertion of the lag screw through the bore in the trochanteric nail; wherein the lag screw further comprises at least one set screw groove defined in the lag screw shaft by a pair of radially inwardly extending, opposing groove sidewalls extending longitudinally at a location on the outer surface of the shaft that is proximal to the thread when the lag screw is oriented for insertion.
11. The system of Example 10, further comprising four of the set screw grooves defined at spaced angular locations on the cylindrical surface of the nail.
12. The system of Example 1, wherein the thread comprises a single start thread.
13. The system of Example 1, wherein the major and minor diameters define a thread height and wherein the undercuts are configured with a radius of curvature selected to extend the undercut across a distance of at least 75% of the thread height.
14. The system of Example 13, wherein the undercut has an outer undercut tip located proximate to the crest of the thread.
15. The system of Example 1, wherein the thread is male.
16. The system of Example 1, wherein the thread is selected from at least one of a right-hand thread and a left-hand thread.
17. A trochanteric nailing system, comprising: a trochanteric nail configured for intramedullary insertion into a femur of a patient to address a hip fracture; and a trochanteric nail fixation device operatively connectable to the trochanteric nail, wherein the trochanteric nail fixation device comprises a lag screw configured to be rotatably advanced into the femur and engage bone matter adjacent to the lag screw in response to torque applied thereto, wherein the lag screw has a head and a tip formed at opposite ends of the lag screw and a shaft extending at least partially between the head and the tip; a helical thread disposed on the outer surface of the shaft, extending longitudinally at a distal location from the head to terminate at the tip; wherein the trochanteric nail further comprises an outer, cylindroid surface characterized by a longitudinal axis and a cross-sectional diameter, the nail having portions defining a bore extending transversely and diametrically through the shaft at an angle to the longitudinal axis, the bore sized to receive the lag screw therethrough, wherein the system further comprises a set screw interconnectable between the nail and the screw, wherein the lag screw is operatively connectable to the trochanteric nail by locking of the set screw to the lag screw after insertion of the lag screw through the bore in the trochanteric nail; wherein the lag screw further comprises four set screw grooves defined in the lag screw shaft by respective pairs of radially inwardly extending, opposing groove sidewalls extending longitudinally at a location on the outer surface of the shaft that is proximal to the thread when the lag screw is oriented for insertion; wherein the pair of groove sidewalls taper in the distal direction to increase engagement with opposing portions of the set screw when received in a selected on of the set screw grooves.
18. A trochanteric nailing system, comprising: a trochanteric nail configured for intramedullary insertion into a femur of a patient to address a hip fracture; and a trochanteric nail fixation device operatively connectable to the trochanteric nail, wherein the trochanteric nail fixation device comprises a lag screw configured to be rotatably advanced into the femur and engage bone matter adjacent to the lag screw in response to torque applied thereto, wherein the lag screw has a head and a tip formed at opposite ends of the lag screw and a shaft extending at least partially between the head and the tip; and a helical thread disposed on the outer surface of the shaft, extending longitudinally at a distal location from the head to terminate at the tip, wherein the thread comprises a threadform having a centerline and a variable cross-sectional width relative to the centerline over adjacent courses of the thread, the width increasing from the tip toward the head of the shaft to increase compression of the bone matter during insertion of the lag screw.
19. The system of claim 18, wherein the thread has a root located radially proximal to the outer surface of the shaft, the thread further having thread sides extending laterally from the root to terminate in a thread crest, whereby the root and the thread crest correspond to the minor and major diameters of the thread, the thread sides corresponding, respectively, to leading and trailing edges of the thread; wherein the thread comprises a threadform having two concavities defined therein, one of the concavities on each of the lateral sides of the threadform to create two, respective undercuts on each of the leading edge and the trailing edge; wherein the thread is characterized by a predetermined pitch selected relative to the bone matter to enable engagement of the bone matter adjacent the lag screw by the undercut, whereby, in response to a lateral force in a given force direction on the lag screw after insertion into the femur, the bone matter engages in portions of the undercut located opposite the given force direction to resist the lateral force; wherein the thread is located to extend longitudinally on the shaft over a predetermined surgical insertion length less than the length of the shaft between the head and the tip of the shaft, and the undercuts extend on the helical thread over the predetermined surgical length; wherein the surgical length and the thread extending over the surgical length define a lateral profile oriented to oppose the lateral force when applied to the lateral profile; wherein the concavity of the undercut defines an angle of at least 45 degrees measured from an associated reference triangular threadform to increase the surface area of the lateral profile over which the lateral force is distributed. wherein the threadform is characterized by a first pitch, and wherein the lag screw further comprises at least one flute extending helically over the surgical length over which the thread extends, the flute having a second pitch, the second pitch being a coarser pitch than the first pitch of the thread to define channels extending through respective thread sides at corresponding radial locations which differ between adjacent courses of the thread.

## Claims

1. A trochanteric nailing system, comprising:
a trochanteric nail configured for intramedullary insertion into a femur of a patient to address a hip fracture; and
a trochanteric nail fixation device operatively connectable to the trochanteric nail, wherein the trochanteric nail fixation device comprises a lag screw configured to be rotatably advanced into the femur and engage bone matter adjacent to the lag screw in response to torque applied thereto, wherein the lag screw has a head and a tip formed at opposite ends of the lag screw and a shaft extending at least partially between the head and the tip;
a helical thread disposed on the outer surface of the shaft, extending longitudinally at a distal location from the head to terminate at the tip, the thread having a root located radially proximal to the outer surface of the shaft, the thread further having thread sides extending laterally from the root to terminate in a thread crest, whereby the root and the thread crest corresponding to the minor and major diameters of the thread, the thread sides corresponding, respectively, to leading and trailing edges of the thread;
wherein the thread comprises a threadform having a concavity defined in at least one of the leading edge and the trailing edge to form at least one, corresponding undercut;
wherein the thread is **characterized by** a predetermined pitch to enable engagement of the bone matter adjacent the lag screw by the undercut, whereby, in response to a lateral force in a given force direction on the lag screw after insertion into the femur, the bone matter engages in portions of the undercut located opposite the given force direction to resist the lateral force.

2. The system of claim 1, wherein the threadform comprises two concavities, one on each of the lateral sides of the threadform to create two, respective undercuts on each of the leading edge and the trailing edge.

3. The system of claim 1 or 2, wherein the thread is located to extend longitudinally on the shaft over a predetermined surgical insertion length less than the length of the shaft between the head and the tip, and the undercuts extend on the helical thread over the predetermined surgical length.

4. The system of any one of the preceding claims, wherein the surgical length and the thread extending over the surgical length define a lateral profile oriented to oppose the lateral force when applied to the lateral profile.

5. The system of any one of the preceding claims, wherein the threadform comprises a centerline and comprises a variable cross-sectional width over adjacent courses of the thread, the width increasing from the tip toward the head of the shaft to increase compression of the bone matter during insertion of the lag screw.

6. The system of any one of the preceding claims, wherein the threadform is shaped to have a first pitch for the leading edge and a second pitch for the trailing edge, the first and second pitches selected to differ from each other and selected to cause increased compression relative to the bone matter on insertion of the lag screw.

7. The system of any one of the preceding claims, wherein the threadform is **characterized by** a first pitch, and wherein the lag screw further comprises at least one flute extending helically with a second pitch, the second pitch being a coarser pitch than the first pitch of the thread to define channels extending through respective thread sides at corresponding radial locations which differ between adjacent courses of the thread.

8. The system of claim 7, further comprising three helical flutes having three corresponding flute starts at angular spaced locations about the shaft.

9. The system of claim 8, wherein the three flutes are defined at three, corresponding arcuately spaced locations in a 360 degree turn of the thread.

10. The system of any one of the preceding claims,
wherein the trochanteric nail further comprises an outer, cylindroid surface **characterized by** a longitudinal axis and a cross-sectional diameter, the nail having portions defining a bore extending transversely and diametrically through the shaft at an angle to the longitudinal axis, the bore sized to receive the lag screw therethrough, wherein the system further comprises a set screw interconnectable between the nail and the screw, wherein the lag screw is operatively connectable to the trochanteric nail by locking of the set screw to the lag screw after insertion of the lag screw through the bore in the trochanteric nail;
wherein the lag screw further comprises at least one set screw groove defined in the lag screw shaft by a pair of radially inwardly extending, opposing groove sidewalls extending longitudinally at a location on the outer surface of the shaft that is proximal to the thread when the lag screw is oriented for insertion.

11. The system of claim 10, further comprising four of the set screw grooves defined at spaced angular locations on the cylindrical surface of the nail.

12. The system of any one of the preceding claims, wherein the thread comprises a single start thread.

13. The system of any one of the preceding claims, wherein the major and minor diameters define a thread height and wherein the undercuts are configured with a radius of curvature selected to extend the undercut across a distance of at least 75% of the thread height.

14. The system of claim 13, wherein the undercut has an outer undercut tip located proximate to the crest of the thread.

15. The system of any one of the preceding claims, wherein the thread is male and, wherein the thread is preferably selected from at least one of a right-hand thread and a left-hand thread.
